# EUROPEAN PATENT APPLICATION

(11) **EP 4 183 859 A1**
(43) Date of publication of application: **24.05.2023**
(21) Application number: 21843137.7
(22) Date of filing: 07.07.2021
(51) Int. Cl.: C11D 17/06, C11D 17/08, C11D 3/386, C12N 15/31, C12N 15/56, C12N 9/26, C12N 9/28

(54) **AMYLASE-INCORPORATED CLEANING AGENT COMPOSITION**

(30) Priority: 15.07.2020 JP 2020121626; 06.10.2020 JP 2020169203; 19.05.2021 JP 2021084329
(71) Applicant: Kao Corporation, Chuo-ku Tokyo 103-8210 (JP)
(72) Inventor: HIOKI, Takahiro, Wakayama-shi, Wakayama 640-8580 (JP); KAWAHARA, Akihito, Wakayama-shi, Wakayama 640-8580 (JP)
(74) Representative: Vossius & Partner Patentanwälte Rechtsanwälte mbB
(86) International application number: PCT/JP2021/025574
(87) International publication number: WO 2022/014428

(57) **Abstract**

Provided is a cleaning composition containing α-amylase which exhibits high specific activity at low temperatures. The cleaning composition comprises one or more proteins selected from the group consisting of the following (A), (B), (C), and (D): (A) a protein consisting of an amino acid sequence having at least 90% identity to the amino acid sequence of SEQ ID NO: 2, and having α-amylase activity; (B) a protein consisting of an amino acid sequence having at least 90% identity to the amino acid sequence of SEQ ID NO: 4, and having α-amylase activity; (C) a protein consisting of an amino acid sequence having at least 90% identity to the amino acid sequence of SEQ ID NO: 6, and having α-amylase activity; and (D) a protein consisting of an amino acid sequence having at least 90% identity to the amino acid sequence of SEQ ID NO: 8, and having α-amylase activity.

## Description

### Field of the Invention

The present invention relates to a cleaning composition containing α-amylase.

### Background of the Invention

α-Amylases are used in a wide range of industries, such as starch, brewing, textiles, pharmaceuticals, and food, are known to have suitability for containing in cleaning agents, and are incorporated into dishwashing cleaning agents for automatic dishwashers and clothing cleaning agents as components removing starch stains.

Known α-amylases useful for cleaning agents are Bacillus sp. KSM-1378 (FERM BP-3048) strain-derived α-amylase AP1378 (Patent Literature 1), Termamyl and Duramyl (registered trademarks), which are *Bacillus licheniformis-derived* α-amylases, Bacillus sp. DSM12649 strain-derived α-amylase AA560 (Patent Literature 2), Bacillus sp. SP722 strain-derived α-amylase SP722 (SEQ ID NO: 4 of Patent Literature 3), Cytophaga-derived α-amylase CspAmy2 (Patent Literature 4), and the like.

In the meantime, in recent years, from the viewpoint of environmental protection and cleaning cost reduction, it is important to reduce temperatures in dishwashing and laundry washing, particularly in laundry washing in laundries. In addition, shortening of the cleaning time is also desired.

However, optimal temperatures of most enzymes, including amylases, are higher than temperatures generally set for low-temperature cleaning. For this reason, it is difficult to completely remove many starch stains. It is important to find α-amylases maintaining cleaning performance and amylolytic activity even at low temperatures, and having a high stain removal effect.

It has been reported that the cleaning performance of α-amylases at low temperatures is inversely correlated with amylase binding to starch (starch absorption), and that amylases with low starch absorption have high cleaning performance at low temperatures (Patent Literature 5). Patent Literature 5 discloses that starch absorption can be reduced by introducing mutations into a known starch-binding residue and its adjacent residue.

[Patent Literature 1] WO 94/26881
[Patent Literature 2] WO 00/60060
[Patent Literature 3] WO 06/002643
[Patent Literature 4] WO 2014/164777
[Patent Literature 5] JP-B-6339499

### Summary of the Invention

### Detailed Description of the Invention

The present invention relates to the following 1) and 2):
1) A cleaning composition comprising one or more proteins selected from the group consisting of the following (A), (B), (C), and (D):
   (A) a protein consisting of an amino acid sequence having at least 90% identity to the amino acid sequence of SEQ ID NO: 2, and having α-amylase activity;
   (B) a protein consisting of an amino acid sequence having at least 90% identity to the amino acid sequence of SEQ ID NO: 4, and having α-amylase activity;
   (C) a protein consisting of an amino acid sequence having at least 90% identity to the amino acid sequence of SEQ ID NO: 6, and having α-amylase activity; and
   (D) a protein consisting of an amino acid sequence having at least 90% identity to the amino acid sequence of SEQ ID NO: 8, and having α-amylase activity.
2) A protein selected from the group consisting of the following (A'), (B'), (C'), and (D'):
   (A') a protein consisting of an amino acid sequence having at least 90% identity to the amino acid sequence of SEQ ID NO: 2, having α-amylase activity, and having deletion of amino acid residues at two or more positions selected from the group consisting of positions R178, G179, T180, and G181 of the amino acid sequence of SEQ ID NO: 2;
   (B') a protein consisting of an amino acid sequence having at least 90% identity to the amino acid sequence of SEQ ID NO: 4, having α-amylase activity, and having deletion of amino acid residues at two or more positions selected from the group consisting of positions R178, G179, T180, and G181 of the amino acid sequence of SEQ ID NO: 4;
   (C') a protein consisting of an amino acid sequence having at least 90% identity to the amino acid sequence of SEQ ID NO: 6, having α-amylase activity, and having deletion of amino acid residues at two or more positions selected from the group consisting of positions R178, G179, T180, and G181 of the amino acid sequence of SEQ ID NO: 6; and
   (D') a protein consisting of an amino acid sequence having at least 90% identity to the amino acid sequence of SEQ ID NO: 8, having α-amylase activity, and having deletion of amino acid residues at two or more positions selected from the group consisting of positions R178, G179, T180, and G181 of the amino acid sequence of SEQ ID NO: 8.

### Brief Description of Drawings

[Fig. 1] Fig. 1 shows the amylolytic activity of each amylase at 20°C and 30°C.
[Fig. 2] Fig. 2 is a molecular phylogenetic tree showing the evolutionary relationship between the amylases of the present invention and existing amylases for a cleaning agent.
[Fig. 3] Fig. 3 shows the detergency of each amylase at 20°C.
[Fig. 4] Fig. 4 shows the amylolytic activity of the amylases of the present invention (YR288 mutants) at 20°C.
[Fig. 5] Fig. 5 shows the detergency of the amylases of the present invention (YR288 mutants) at 20°C.
[Fig. 6] Fig. 6 shows the amylolytic activity of the amylases of the present invention (BCGAmy mutants) at 20°C.
[Fig. 7] Fig. 7 shows the cleaning performance of a cleaning composition containing protease (detergency against CS-26 stained cloth).
[Fig. 8] Fig. 8 shows the cleaning performance of a cleaning composition containing protease (detergency against EMPA117 stained cloth).

For the exhibition of high cleaning performance at low temperatures, two points, i.e., maintenance of amylolytic activity at low temperatures and low starch absorption, are considered to be particularly important; however, it is not easy to maintain amylolytic activity at low temperatures. Conventionally, none of existing amylases for a cleaning agent sufficiently maintains amylolytic activity at low temperatures, despite the fact that the strength of activity has been considered as part of the screening criteria.

Therefore, in order to develop amylases exhibiting higher detergency at low temperatures in comparison with the existing detergency amylases, it is important to newly find amylases holding high amylolytic activity at low temperatures. However, there is no sequence index for screening amylases holding higher amylolytic activity at low temperatures than the existing amylases, and it is not easy to search for such amylases.

Since many stains containing starch also contain protein components, it is known that the combined use of α-amylase and protease produces an additive/ synergistic cleaning effect. Cleaning agents containing α-amylase and protease are particularly useful for low-temperature cleaning where reduced detergency is an issue. Maintaining amylase activity at low temperatures is still a major issue for such cleaning agents. None of the existing amylases for a cleaning agent sufficiently maintains amylolytic activity at low temperatures.

The present invention relates to the provision of cleaning compositions containing α-amylases which exhibit high specific activity (amylolytic activity) at low temperatures.

The present inventors found that specific α-amylases found from estimated α-amylase sequences included in the NCBI protein sequence database have high amylolytic activity at a low temperature of from 20 to 30°C, and that cleaning compositions containing such α-amylases are useful as cleaning compositions suitable for low-temperature cleaning.

The present invention can provide a cleaning composition which exhibits an excellent starch stain removal effect even when used for low-temperature cleaning. The sequence information of the protein of the present invention can be used as a sequence index for screening α-amylases having high amylolytic activity at low temperatures.

In the present invention, the identity of nucleotide sequences and amino acid sequences is calculated by the Lipman-Pearson method (Science, 1985, 227: 1435-1441). Specifically, the identity is calculated by analysis using a homology analysis (Search homology) program of genetic information processing software Genetyx-Win at a unit size to compare (ktup) of 2.

In the present invention, "at least 90% identity" relating to amino acid sequences or nucleotide sequences refers to 90% or more, preferably 95% or more, more preferably 97% or more, even more preferably 98% or more, and even more preferably 99% or more identity.

The amino acid sequences having at least 90% identity include amino acid sequences having deletion, insertion, substitution, or addition of one or several amino acids. Examples of the "amino acid sequences having deletion, insertion, substitution, or addition of one or several amino acids" include amino acid sequences having deletion, insertion, substitution, or addition of 1 or more and 30 or less, preferably 20 or less, more preferably 10 or less, and even more preferably 5 or less amino acids.

The protein contained in the cleaning composition of the present invention is one or more proteins selected from the group consisting of the following (A), (B), (C), and (D):
(A) a protein consisting of an amino acid sequence having at least 90% identity to the amino acid sequence of SEQ ID NO: 2, and having α-amylase activity;
(B) a protein consisting of an amino acid sequence having at least 90% identity to the amino acid sequence of SEQ ID NO: 4, and having α-amylase activity;
(C) a protein consisting of an amino acid sequence having at least 90% identity to the amino acid sequence of SEQ ID NO: 6, and having α-amylase activity; and
(D) a protein consisting of an amino acid sequence having at least 90% identity to the amino acid sequence of SEQ ID NO: 8, and having α-amylase activity.

These proteins have excellent α-amylase activity at from 20 to 30°C.

The α-amylase activity as mentioned herein means an activity that catalyzes the hydrolysis of starch and other linear or branched 1,4-glycoside oligosaccharides or polysaccharides.

The α-amylase activity can be determined by measuring the amount of reducing ends produced by the enzymatic degradation of starch. The determination method is not limited thereto; for example, the α-amylase activity can also be determined by measuring the release of dye by the enzymatic degradation of dye-crosslinked starch, such as Phadebas (Soininen, K., M. Ceska, and H. Adlercreutz. "Comparison between a new chromogenic α-amylase test (Phadebas) and the Wohlgemuth amyloclastic method in urine." Scandinavian journal of clinical and laboratory investigation 30.3 (1972): 291-297.). There is a correlation between the α-amylase activity measured using Phadebas and the cleaning performance when used as a cleaning agent.

The proteins consisting of the amino acid sequences of SEQ ID NOs: 2, 4, 6, and 8 are proteins estimated to be α-amylases in the NCBI protein sequence database. Specifically, in this database, the protein consisting of the amino acid sequence of SEQ ID NO: 2 is registered as accession number WP_138117433.1 (referred to as "DE0178" in the present invention), the protein consisting of the amino acid sequence of SEQ ID NO: 4 as WP_076512862.1 (referred to as "RU2C" in the present invention), the protein consisting of the amino acid sequence of SEQ ID NO: 6 as WP_110114708.1 (referred to as "BCGAmy" in the present invention), and the protein consisting of the amino acid sequence of SEQ ID NO: 8 as WP_100346362.1 (referred to as "YR288" in the present invention). However, their enzymological properties have not been reported at all.

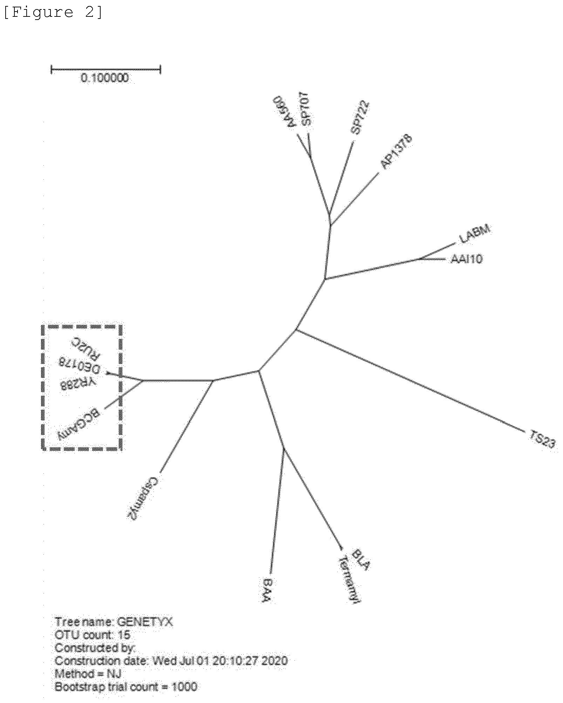

In order to clarify the evolutionary relationship between DE0178, RU2C, BCGAmy, and YR288 mentioned above, and AP1378 (SEQ ID NO: 10), AA560 (SEQ ID NO: 12), SP722 (SEQ ID NO: 14), CspAmy2 (SEQ ID NO: 16), BAA (SEQ ID NO: 35), BLA (SEQ ID NO: 36), LABM (SEQ ID NO: 37), SP707 (SEQ ID NO: 38), TS23 (SEQ ID NO: 39), Termamyl (SEQ ID NO: 40), and AAI10 (SEQ ID NO: 41), which are known as amylases for a cleaning agent, a molecular phylogenetic tree was constructed by the neighbor-joining method using the amino acid sequence of the mature region of each amylase. As a result, it was indicated that DE0178, RU2C, BCGAmy, and YR288 of the present invention belong to different groups from the existing amylases for a cleaning agent (Fig. 2).

As one aspect, the proteins consisting of amino acid sequences having at least 90% identity to the amino acid sequences of SEQ ID NOs: 2, 4, 6, and 8, and having α-amylase activity include protein mutants consisting of the amino acid sequences of SEQ ID NOs: 2, 4, 6, and 8, but having substitution, deletion, insertion, or addition of one or several amino acids, and having α-amylase activity. Examples of the number of "several amino acids" include from 2 to 10, preferably from 2 to 5, and more preferably 2 or 3.

Among such mutants, preferred in terms of amylolytic activity and/or cleaning properties at low temperatures are mutants consisting of the amino acid sequences set forth in SEQ ID NOs: 2, 4, 6, and 8 but having deletion of amino acid residues at two or more positions selected from the group consisting of arginine at position 178 (R178), glycine at position 179 (G179), threonine at position 180 (T180), and glycine at position 181 (G181); and more preferred are mutants consisting of the amino acid sequence of SEQ ID NO: 8 but having deletion of amino acid residues at two or more positions selected from the group consisting of R178, G179, T180, and G181.

Examples of the deletion of amino acid residues at two or more positions (expressed by [original amino acid, position, Δ]) preferably include R178Δ+T180Δ, G179Δ+T180Δ, R178Δ+G179Δ, R178Δ+G181Δ, G179Δ+G181Δ, and the like; and more preferably R178Δ+T180Δ.

The above mutants are novel proteins which have not reported in literatures. Therefore, as one aspect, the present invention includes a protein selected from the group consisting of the following (A'), (B'), (C'), and (D'), and a cleaning composition containing the protein:
(A') a protein consisting of an amino acid sequence having at least 90% identity to the amino acid sequence of SEQ ID NO: 2, having α-amylase activity, and having deletion of amino acid residues at two or more positions selected from the group consisting of positions R178, G179, T180, and G181 of the amino acid sequence of SEQ ID NO: 2;
(B') a protein consisting of an amino acid sequence having at least 90% identity to the amino acid sequence of SEQ ID NO: 4, having α-amylase activity, and having deletion of amino acid residues at two or more positions selected from the group consisting of positions R178, G179, T180, and G181 of the amino acid sequence of SEQ ID NO: 4;
(C') a protein consisting of an amino acid sequence having at least 90% identity to the amino acid sequence of SEQ ID NO: 6, having α-amylase activity, and having deletion of amino acid residues at two or more positions selected from the group consisting of positions R178, G179, T180, and G181 of the amino acid sequence of SEQ ID NO: 6; and
(D') a protein consisting of an amino acid sequence having at least 90% identity to the amino acid sequence of SEQ ID NO: 8, having α-amylase activity, and having deletion of amino acid residues at two or more positions selected from the group consisting of positions R178, G179, T180, and G181 of the amino acid sequence set of SEQ ID NO: 8.

The protein of the present invention can be produced, for example, by expressing a gene encoding the protein of the present invention.

Preferably, the protein of the present invention can be produced from a transformant into which a polynucleotide encoding the protein of the present invention is introduced. For example, a polynucleotide encoding the protein of the present invention, or a vector containing the polynucleotide, is introduced into a host to obtain a transformant, and the transformant is then cultured in a suitable culture medium, whereby the protein of the present invention is produced from the polynucleotide encoding the protein of the present invention introduced into the transformant. The produced protein can be isolated or purified from the culture to thereby obtain the protein of the present invention.

The polynucleotide encoding the protein of the present invention can be in the form of single- or double-stranded DNA, RNA, or an artificial nucleic acid, or may be cDNA or chemically synthesized intron-free DNA. Preferred examples of the polynucleotide encoding the protein of the present invention include the following polynucleotides (a), (b), (c), and (d):
(a) a polynucleotide consisting of a base sequence having at least 90% identity to the base sequence of SEQ ID NO: 1;
(b) a polynucleotide consisting of a base sequence having at least 90% identity to the base sequence of SEQ ID NO: 3;
(c) a polynucleotide consisting of a base sequence having at least 90% identity to the base sequence of SEQ ID NO: 5; and
(d) a polynucleotide consisting of a base sequence having at least 90% identity to the base sequence of SEQ ID NO: 7.

The polynucleotide encoding the protein of the present invention can be synthesized chemically or by genetic engineering based on the amino acid sequence of the protein. For example, the polynucleotide can be chemically synthesized based on the amino acid sequence of the protein of the present invention described above. For the chemical synthesis of the polynucleotide, contracted synthesis service of nucleic acids (e.g., available from Medical & Biological Laboratories Co., Ltd., GenScript, and the like) can be used. Further, the synthesized polynucleotide can be amplified by PCR, cloning, or the like.

As the polynucleotide encoding the protein, which is a mutant, a polynucleotide encoding the mutated amino acid is produced by using various mutagenesis techniques known in this technical field. Introduction of mutations can be basically performed by various site-specific mutagenesis methods well-known to a person skilled in the art. The site-specific mutagenesis method can be performed by any method, such as an inverse PCR method or an annealing method. It is also possible to use commercially available site-specific mutagenesis kits (e.g., Stratagene's QuickChange II Site-Directed Mutagenesis Kit and QuickChange Multi Site-Directed Mutagenesis Kit, or the like).

Most commonly, site-specific mutagenesis can be performed by using a mutation primer containing the nucleotide mutation to be introduced. The mutation primer may be designed to be annealed to a region containing a nucleotide sequence encoding an amino acid residue to be mutated in a polynucleotide encoding a parent protein, and to contain a nucleotide sequence having a nucleotide sequence (codon) encoding the mutated amino acid residue in place of the nucleotide sequence (codon) encoding the amino acid residue to be mutated. The nucleotide sequences (codons) encoding the unmutated or mutated amino acid residues can be appropriately recognized and selected by a person skilled in the art based on ordinary textbooks and the like. Alternatively, site-specific mutagenesis can also be performed by using a method in which DNA fragments, obtained by amplifying the upstream and downstream sides of the mutation site separately using two complementary primers containing the nucleotide mutation to be introduced, are linked into one by SOE (splicing by overlap extension)-PCR (Gene, 1989, 77 (1) : pp. 61-68) .

The type of vector containing the polynucleotide encoding the protein of the present invention is not particularly limited, and any vector, such as a plasmid, phage, phagemid, cosmid, virus, YAC vector, or shuttle vector, may be used. The vector is not limited, but is preferably a vector which can be amplified in bacteria, preferably Bacillus bacteria (e.g., *Bacillus subtilis* or mutant strains thereof), and more preferably an expression vector which can induce the expression of transgenes in Bacillus bacteria. Among these, shuttle vectors, which are vectors replicable in Bacillus bacteria and any other organisms, can be preferably used in the recombinant production of the mutant of the present invention. Examples of preferred vectors include, but are not limited to, pHA3040SP64, pHSP64R, or pASP64 (JP-B-3492935), pHY300PLK (an expression vector which can transform both *Escherichia coli* and *Bacillus subtilis*; Jpn J Genet, 1985, 60: 235-243), pAC3 (Nucleic Acids Res, 1988, 16: 8732), and other shuttle vectors; pUB110 (J Bacteriol, 1978, 134: 318-329), pTA10607 (Plasmid, 1987, 18: 8-15), and other plasmid vectors which can be used in the transformation of Bacillus bacteria; and the like. Other usable examples include *Escherichia coli*-derived plasmid vectors (e.g., pET22b(+), pBR322, pBR325, pUC57, pUC118, pUC119, pUC18, pUC19, and pBluescript, and the like).

The above vector may contain a DNA replication initiation region or a DNA region containing a replication origin. Alternatively, in the above vector, a regulatory sequence, such as a promoter region for initiating the transcription of the gene, a terminator region, or a secretion signal region for secreting the expressed protein outside the cell, may be operably linked to the upstream of the polynucleotide encoding the protein of the present invention (i.e., α-amylase gene). The phrase that a gene and a regulatory sequence are "operably linked" means that the gene and the regulatory region are arranged so that the gene can be expressed under the control of the regulatory region.

The type of regulatory sequence, such as a promoter region, a terminator, or a secretion signal region mentioned above, is not particularly limited, and generally used promoters and secretion signal sequences can be appropriately selected depending on the host for introduction. Examples of preferred regulatory sequences that can be incorporated into the vector include the promoter, secretion signal sequence, and the like of the cellulase gene of Radius sp. KSM-S237 strain.

Alternatively, a marker gene (e.g., a gene resistant to drugs, such as ampicillin, neomycin, kanamycin, and chloramphenicol) for selecting the host into which the vector of the present invention is appropriately introduced may be further incorporated into the vector. Alternatively, when an auxotroph is used as the host, a gene encoding the desired nutritional synthetic enzyme may be incorporated as a marker gene into the vector. Alternatively, when a selective culture medium in which a specific metabolism is required for growth, is used, a gene associated with the metabolism may be incorporated as a marker gene into the vector. Examples of such metabolism-related gene include acetamidase genes for utilizing acetamide as a nitrogen source.

The polynucleotide encoding the protein of the present invention, a regulatory sequence, and a marker gene can be linked by a method known in the art, such as SOE (splicing by overlap extension)-PCR (Gene, 1989, 77: 61-68). Procedures for introducing the linked fragment into the vector are well known in the art.

Examples of the host of the transformant into which the vector is introduced include microorganisms, such as bacteria and filamentous fungi. Examples of bacteria include *Escherichia coli* and bacteria belonging to the genera Staphylococcus, Enterococcus, Listeria, and Bacillus. Preferred among these are *Escherichia coli* and Bacillus bacteria (e.g., *Bacillus subtilis* Marburg No. 168 (*Bacillus subtilis* 168 strain) or mutant strains thereof). Examples of *Bacillus subtilis* mutant strains include the nine-protease-deficient strain KA8AX described in J. Biosci. Bioeng., 2007, 104(2): 135-143, and the eight-protease-deficient strain with improved protein folding efficiency, D8PA strain, described in Biotechnol. Lett., 2011, 33(9): 1847-1852. Examples of filamentous fungi include Trichoderma, Aspergillus, Rizhopus, and the like.

Methods commonly used in the art, such as the protoplast method and the electroporation method, can be used to introduce the vector into the host. Strains with appropriate introduction are selected using marker gene expression, auxotrophy, and the like as indices, whereby the target transformant into which the vector is introduced can be obtained.

Alternatively, a fragment obtained by linking the polynucleotide encoding the protein of the present invention, a regulatory sequence, and a marker gene can also be introduced directly into the genome of the host. For example, a DNA fragment in which sequences complementary to the genome of the host are added to both ends of the linked fragment is constructed by the SOE-PCR method or the like, and this DNA fragment is then introduced into the host to induce homologous recombination between the host genome and the DNA fragment, whereby the polynucleotide encoding the protein of the present invention is introduced into the genome of the host.

When the thus-obtained transformant into which the polynucleotide encoding the protein of the present invention, or a vector containing the polynucleotide is introduced, is cultured in a suitable culture medium, the gene encoding the protein on the vector is expressed to produce the protein of the present invention. The culture medium used for culturing the transformant can be appropriately selected by a person skilled in the art depending on the type of microorganism of the transformant.

Alternatively, the protein of the present invention may be expressed from the polynucleotide encoding the protein of the present invention or a transcript thereof using a cell-free translation system. The "cell-free translation system" is such that reagents, such as amino acids, necessary for the protein translation are added to a suspension obtained by mechanically destroying a cell, which serves as the host, to construct an in vitro transcription-translation system or an in vitro translation system.

The protein of the present invention produced in the above culture or cell-free translation system can be isolated or purified by using general methods used for protein purification, such as centrifugation, ammonium sulfate precipitation, gel chromatography, ion-exchange chromatography, and affinity chromatography, singly or in a suitable combination. In this case, when the gene encoding the protein of the present invention is operably linked to a secretion signal sequence on the vector in the transformant, the produced protein is secreted extracellularly, and can be thus more easily collected from the culture. The protein collected from the culture may be further purified by known means.

The thus-obtained protein of the present invention exhibits significantly higher amylolytic activity at 20°C and 30°C in comparison with the previously reported amylases for a cleaning agent (AP1378, AA560, SP722, and CspAmy2).

The protein of the present invention has superior cleaning performance in comparison with the existing amylases for a cleaning agent. Specifically, when the cleaning performance is measured by the following method, the relative cleaning performance with respect to AA560 (amylase consisting of the amino acid sequence of SEQ ID NO: 12) is 1 or more, preferably 1.1 or more, more preferably 1.2 or more, even more preferably 1.3 or more, even more preferably 1.5 or more, even more preferably 1.7 or more, and even more preferably 1.9 or more.

### <Measurement of cleaning performance>

Two CS-26 stained cloths cut into a circular shape with a diameter of 5.5 mm (CFT) are inserted into each well of a 96-well assay plate, and 200 µL of a model cleaning liquid (200 ppm sodium linear alkylbenzene sulfonate and 20 mM Tris HCl aqueous solution (pH: 7.5)) is added to each well. 10 µL of a test amylase solution diluted to 4 ppm is added to each well, followed by shaking at 1,200 rpm for 15 minutes at 20°C. After the completion of cleaning, the absorbance of the cleaning liquid at 488 nm is measured. A blank is prepared by adding ion-exchange water in place of the enzyme solution, and the difference from the blank ΔA488 is defined as the cleaning performance.

The relative cleaning performance with respect to AA560 is calculated by (ΔA488 of test amylase)/(ΔA488 of AA560).

Therefore, the protein of the present invention is useful as an enzyme to be contained in various cleaning compositions, and particularly useful as an enzyme to be contained in cleaning compositions suitable for low-temperature cleaning.

Examples of the "low temperature" as mentioned herein include 40°C or lower, 35°C or lower, 30°C or lower, and 25°C or lower, and also include 5°C or higher, 10°C or higher, and 15°C or higher. Other examples include from 5 to 40°C, from 10 to 35°C, from 15 to 30°C, and from 15 to 25°C.

The amount of the protein of the present invention contained in the cleaning composition is not particularly limited as long as the protein can exhibit activity. For example, the amount of the protein per kg of the cleaning composition is preferably 1 mg or more, more preferably 10 mg or more, and even more preferably 50 mg or more, as well as preferably 5,000 mg or less, more preferably 1,000 mg or less, and even more preferably 500 mg or less. The amount of the protein is also preferably from 1 to 5,000 mg, more preferably from 10 to 1,000 mg, and even more preferably from 50 to 500 mg.

In the cleaning composition of the present invention, various enzymes other than the protein of the present invention can be used in combination. Examples include hydrolases, oxidases, reductases, transferases, lyases, isomerases, ligases, synthetases, and the like. Preferred among these are amylases which are different from the protein of the present invention, proteases, cellulases, keratinases, esterases, cutinases, lipases, pullulanases, pectinases, mannanases, glucosidases, glucanases, cholesterol oxidases, peroxidases, laccases, and the like; and particularly preferred are proteases, cellulases, amylases, and lipases.

Examples of proteases include proteins having at least 70%, preferably 80% or more, more preferably 90% or more, and even more preferably 95% or more identity to the amino acid sequence of SEQ ID NO: 42, 43, 44, 45, 46, 47, or 48, and having protease activity.

The protease consisting of the amino acid sequence of SEQ ID NO: 42 is *Bacillus clausii*-derived protease Savinase (WO 2011/036263), the protease consisting of the amino acid sequence of SEQ ID NO: 43 is *Bacillus amyloliquefaciens-derived* protease BPN' (WO 2011/036263), the protease consisting of the amino acid sequence of SEQ ID NO: 44 is *Bacillus lentus* DSM 5483-derived protease (WO 92/21760), the protease consisting of the amino acid sequence of SEQ ID NO: 45 is Bacillus sp. KSM-KP43-derived protease (WO 99/18218), the protease consisting of the amino acid sequence of SEQ ID NO: 46 is Bacillus sp.-derived protease TY145 (JP-A-2019-503404), the protease consisting of the amino acid sequence of SEQ ID NO: 47 is *Bacillus amyloliquefacience-derived* protease Neutrase, and the protease consisting of the amino acid sequence of SEQ ID NO: 48 is *Bacillus subtilis-derived* metalloprotease.

Proteases may also be commercially available Alcalase, Esperase, Everlase, Kannase, and Progress Uno (registered trademarks; Novozymes A/S), PREFERENZ, EFFECTENZ, and EXCELLENZ (registered trademarks; DuPont), Lavergy (registered trademark; BASF), and the like.

Examples of cellulases include Celluclean and Carezyme (registered trademarks; Novozymes A/S); KAC, the alkaline cellulase produced by Bacillus sp. KSM-S237 strain described in JP-A-10-313859, and the mutant alkaline cellulase described in JP-A-2003-313592 (Kao Corporation); and the like.

Examples of amylases include Termamyl, Duramyl, Stainzyme, Stainzyme Plus, and Amplify Prime (registered trademarks; Novozymes A/S), PREFERENZ and EFFECTENZ (registered trademarks; DuPont), KAM (Kao Corporation), and the like.

Examples of lipases include Lipolase and Lipex (registered trademarks; Novozymes A/S), and the like.

Known cleaning agent components can be contained in the cleaning composition of the present invention, and examples of such known cleaning agent components include the following.

### (1) Surfactant

A surfactant may be contained in an amount of from 0.5 to 60 mass% in the cleaning composition, and preferably from 10 to 45 mass% particularly in a powder cleaning composition, and from 20 to 90 mass% in a liquid cleaning composition. When the cleaning composition of the present invention is a clothing cleaning agent for laundry or a cleaning agent for an automatic dishwasher, the surfactant is generally contained in an amount of from 1 to 10 mass%, and preferably from 1 to 5 mass%.

Examples of the surfactant used in the cleaning composition of the present invention include one or a combination of anionic surfactants, nonionic surfactants, amphoteric surfactants, and cationic surfactants; and anionic surfactants and nonionic surfactants are preferred.

Examples of preferred anionic surfactants include sulfate ester salts of alcohols having from 10 to 18 carbon atoms, sulfate ester salts of alkoxylated alcohols having from 8 to 20 carbon atoms, alkylbenzene sulfonate, paraffin sulfonate, α-olefin sulfonate, internal olefin sulfonate, α-sulfo fatty acid salts, α-sulfo fatty acid alkyl ester salts, and fatty acid salts. In the present invention, particularly preferred is one or more anionic surfactants selected from the group consisting of linear alkylbenzene sulfonate with an alkyl chain having from 10 to 14 carbon atoms, more preferably from 12 to 14 carbon atoms, and internal olefin sulfonate with an alkylene chain having from 12 to 20 carbon atoms, more preferably from 16 to 18 carbon atoms. Alkali metal salts and amines are preferable as counterions, and sodium and/or potassium, monoethanolamine, and diethanolamine are particularly preferred. For internal olefin sulfonic acid, reference can be made to, for example, WO 2017/098637.

Preferred nonionic surfactants are polyoxyalkylene alkyl (from 8 to 20 carbon atoms) ether, alkyl polyglycoside, polyoxyalkylene alkyl (from 8 to 20 carbon atoms) phenyl ether, polyoxyalkylene sorbitan fatty acid (from 8 to 22 carbon atoms) ester, polyoxyalkylene glycol fatty acid (from 8 to 22 carbon atoms) ester, and polyoxyethylene polyoxypropylene block polymers. In particular, preferred nonionic surfactants are polyoxyalkylene alkyl ethers obtained by adding 4 to 20 moles of alkylene oxides, such as ethylene oxide and propylene oxide, to alcohols having from 10 to 18 carbon atoms [an HLB value (calculated by the Griffin method) of from 10.5 to 15.0, and preferably from 11.0 to 14.5].

### (2) Divalent metal ion scavenger

A divalent metal ion scavenger may be contained in an amount of from 0.01 to 50 mass%, and preferably from 5 to 40 mass%. Examples of the divalent metal ion scavenger used in the cleaning composition of the present invention include condensed phosphates, such as tripolyphosphates, pyrophosphates, and orthophosphates; aluminosilicates, such as zeolites; synthetic layered crystalline silicates, nitrilotriacetates, ethylenediaminetetraacetates, citrates, isocitrates, polyacetal carboxylates, and the like. Among these, crystalline aluminosilicates (synthetic zeolites) are particularly preferred. Among A-, X-, and P-type zeolites, A-type zeolites are particularly preferred. As synthetic zeolites, those having an average primary particle size of from 0.1 to 10 µm, particularly from 0.1 to 5 µm, are preferably used.

### (3) Alkali agent

An alkali agent may be contained in an amount of from 0.01 to 80 mass%, preferably from 1 to 40 mass%. In the case of powder cleaning agents, examples of alkali agents include alkali metal carbonates, such as sodium carbonate, collectively called dense ash or light ash; and amorphous alkali metal silicates, such as JIS Nos. 1, 2, and 3. These inorganic alkali agents are effective in the formation of the particle skeleton during drying of cleaning agent, and relatively hard cleaning agents having excellent flowability can be obtained. Examples of other alkalis include sodium sesquicarbonate, sodium hydrogencarbonate, and the like. In addition, phosphates, such as tripolyphosphates, also have the action as alkali agents. As alkali agents used in liquid cleaning agents, sodium hydroxide and mono-, di-, or triethanolamine can be used, in addition to the alkali agents mentioned above, and they can also be used as counterions of activators.

### (4) Anti-redeposition agent

The anti-redeposition agent may be contained in an amount of from 0.001 to 10 mass%, preferably from 1 to 5 mass%. Examples of the anti-redeposition agent used in the cleaning composition of the present invention include polyethylene glycol, carboxylic acid-based polymers, polyvinyl alcohol, polyvinylpyrrolidone, and the like. Among these, carboxylic acid-based polymers have the function of scavenging metal ions and the ability to disperse solid particle stains from the clothing into the laundry bath, as well as the anti-redeposition ability. Carboxylic acid-based polymers are homopolymers or copolymers of acrylic acid, methacrylic acid, itaconic acid, or the like. Preferred copolymers are copolymers of the above monomers and maleic acid, and those with a molecular weight of from several thousands to a hundred thousand are preferred. In addition to the carboxylic acid-based polymers mentioned above, polymers such as polyglycidylates, cellulose derivatives such as carboxymethyl cellulose, and amino carboxylic acid-based polymers such as polyaspartic acid are also preferred because they have metal ion scavenging, dispersion, and anti-redeposition ability.

### (5) Bleaching agent

A bleaching agent, such as hydrogen peroxide or percarbonate, may be preferably contained in an amount of from 1 to 10 mass%. When the bleaching agent is used, tetraacetylethylenediamine (TAED) or the bleach activator described in JP-A-6-316700 can be contained in an amount of from 0.01 to 10 mass%.

### (6) Fluorescent agent

Examples of the fluorescent agent used in the cleaning composition of the present invention include biphenyl-type fluorescent agents (e.g., Tinopal CBS-X and the like) and stilbene-type fluorescent agents (e.g., a DM-type fluorescent dye and the like). The fluorescent agent is preferably contained in an amount of from 0.001 to 2 mass%.

### (7) Other components

The cleaning composition of the present invention may contain builders, softeners, reducing agents (e.g., sulfite), foam inhibitors (e.g., silicone), fragrances, antibacterial and antifungal agents (e.g., Proxel [trade name] and benzoic acid), and other additives known in the field of clothing cleaning agents.

The cleaning composition of the present invention can be produced in accordance with a standard method by combining the protein of the present invention obtained by the above method and the known cleaning components mentioned above. The form of the cleaning agent can be selected depending on the application. For example, the cleaning agent can be in the form of liquid, powder, granules, paste, solids, or the like.

The thus-obtained cleaning composition of the present invention can be used as a clothing cleaning agent, a dishwashing cleaning agent, a bleaching agent, a cleaning agent for hard surface cleaning, a drain cleaning agent, a denture cleaning agent, a disinfecting cleaning agent for medical instruments, or the like; preferably a clothing cleaning agent and a dishwashing cleaning agent; and more preferably a clothing cleaning agent for laundry (laundry cleaning agent), a dishwashing cleaning agent for hand washing, and a cleaning agent for an automatic dishwasher.

The cleaning composition is suitable for use at 40°C or lower, 35°C or lower, 30°C or lower, or 25°C or lower, and at 5°C or higher, 10°C or higher, or 15°C or higher. The cleaning composition is also suitable for use at from 5 to 40°C, from 10 to 35°C, from 15 to 30°C, or from 15 to 25°C. Preferred use modes include use for low-temperature (from 15 to 30°C) cleaning in laundries, and low-temperature (from 15 to 30°C) cleaning by the automatic dishwasher.

Regarding the embodiments described above, the present invention further discloses the following aspects.
<1> A cleaning composition comprising one or more proteins selected from the group consisting of the following (A), (B), (C), and (D):
   (A) a protein consisting of an amino acid sequence having at least 90% identity to the amino acid sequence of SEQ ID NO: 2, and having α-amylase activity;
   (B) a protein consisting of an amino acid sequence having at least 90% identity to the amino acid sequence of SEQ ID NO: 4, and having α-amylase activity;
   (C) a protein consisting of an amino acid sequence having at least 90% identity to the amino acid sequence of SEQ ID NO: 6, and having α-amylase activity; and
   (D) a protein consisting of an amino acid sequence having at least 90% identity to the amino acid sequence of SEQ ID NO: 8, and having α-amylase activity.
<2> The cleaning composition according to <1>, wherein the protein is one or more selected from the group consisting of the following (A'), (B'), (C'), and (D'):
   (A') a protein consisting of an amino acid sequence having at least 90% identity to the amino acid sequence of SEQ ID NO: 2, having α-amylase activity, and having deletion of amino acid residues at two or more positions selected from the group consisting of positions R178, G179, T180, and G181 of the amino acid sequence of SEQ ID NO: 2;
   (B') a protein consisting of an amino acid sequence having at least 90% identity to the amino acid sequence of SEQ ID NO: 4, having α-amylase activity, and having deletion of amino acid residues at two or more positions selected from the group consisting of positions R178, G179, T180, and G181 of the amino acid sequence of SEQ ID NO: 4;
   (C') a protein consisting of an amino acid sequence having at least 90% identity to the amino acid sequence of SEQ ID NO: 6, having α-amylase activity, and having deletion of amino acid residues at two or more positions selected from the group consisting of positions R178, G179, T180, and G181 of the amino acid sequence of SEQ ID NO: 6; and
   (D') a protein consisting of an amino acid sequence having at least 90% identity to the amino acid sequence of SEQ ID NO: 8, having α-amylase activity, and having deletion of amino acid residues at two or more positions selected from the group consisting of positions R178, G179, T180, and G181 of the amino acid sequence of SEQ ID NO: 8.
<3> The cleaning composition according to <1>,
   wherein the protein is a protein having a relative cleaning performance of 1 or more, preferably 1.1 or more, more preferably 1.2 or more, even more preferably 1.3 or more, even more preferably 1.5 or more, even more preferably 1.7 or more, and even more preferably 1.9 or more, with respect to an amylase consisting of the amino acid sequence of SEQ ID NO: 12.
<4> The cleaning composition according to any one of <1> to <3>, which is a clothing cleaning agent or a dishwashing cleaning agent.
<5> The cleaning composition according to <4>, which is a clothing cleaning agent for laundry or a dishwashing cleaning agent for hand washing or an automatic dishwasher.
<6> The cleaning composition according to <4> or <5>, which is a powder or a liquid.
<7> The cleaning composition according to any one of <4> to <6>, which is used at a low temperature.
<8> The cleaning composition according to <7>, which is used at 40°C or lower, 35°C or lower, 30°C or lower, or 25°C or lower, and 5°C or higher, 10°C or higher, or 15°C or higher, or used at from 5 to 40°C, from 10 to 35°C, from 15 to 30°C, or from 15 to 25°C.
<9> The cleaning composition according to <4>, which is used in low-temperature (from 15 to 30°C) cleaning in laundries or low-temperature (from 15 to 30°C) cleaning by the automatic dishwasher.
<10> A protein selected from the group consisting of the following (A'), (B'), (C'), and (D'):
   (A') a protein consisting of an amino acid sequence having at least 90% identity to the amino acid sequence of SEQ ID NO: 2, having α-amylase activity, and having deletion of amino acid residues at two or more positions selected from the group consisting of positions R178, G179, T180, and G181 of the amino acid sequence of SEQ ID NO: 2;
   (B') a protein consisting of an amino acid sequence having at least 90% identity to the amino acid sequence of SEQ ID NO: 4, having α-amylase activity, and having deletion of amino acid residues at two or more positions selected from the group consisting of positions R178, G179, T180, and G181 of the amino acid sequence of SEQ ID NO: 4;
   (C') a protein consisting of an amino acid sequence having at least 90% identity to the amino acid sequence of SEQ ID NO: 6, having α-amylase activity, and having deletion of amino acid residues at two or more positions selected from the group consisting of positions R178, G179, T180, and G181 of the amino acid sequence of SEQ ID NO: 6; and
   (D') a protein consisting of an amino acid sequence having at least 90% identity to the amino acid sequence of SEQ ID NO: 8, having α-amylase activity, and having deletion of amino acid residues at two or more positions selected from the group consisting of positions R178, G179, T180, and G181 of the amino acid sequence of SEQ ID NO: 8.
<11> The protein according to <10>, which has a relative cleaning performance of 1 or more, preferably 1.1 or more, more preferably 1.2 or more, even more preferably 1.3 or more, even more preferably 1.5 or more, even more preferably 1.7 or more, and even more preferably 1.9 or more, with respect to an amylase consisting of the amino acid sequence of SEQ ID NO: 12.
<12> The protein according to <10> or <11>, or the cleaning composition according to <2> or <3>, wherein the deletion of amino acid residues at two or more positions selected from the group consisting of positions R178, G179, T180, and G181 is R178Δ+T180Δ, G179Δ+T180Δ, R178Δ+G179Δ, R178Δ+G181Δ, or G179Δ+G181Δ, and preferably R178Δ+T180Δ.
<13> A cleaning composition comprising one or more proteins having α-amylase activity selected from the group consisting of the following (A), (B), (C), and (D), and a protein having protease activity:
   (A) a protein consisting of an amino acid sequence having at least 90% identity to the amino acid sequence of SEQ ID NO: 2, and having α-amylase activity;
   (B) a protein consisting of an amino acid sequence having at least 90% identity to the amino acid sequence of SEQ ID NO: 4, and having α-amylase activity;
   (C) a protein consisting of an amino acid sequence having at least 90% identity to the amino acid sequence of SEQ ID NO: 6, and having α-amylase activity; and
   (D) a protein consisting of an amino acid sequence having at least 90% identity to the amino acid sequence of SEQ ID NO: 8, and having α-amylase activity.
<14> The cleaning composition according to <13>, wherein the protein having α-amylase activity is one or more members selected from the group consisting of the following (A'), (B'), (C'), and (D'):
   (A') a protein consisting of an amino acid sequence having at least 90% identity to the amino acid sequence of SEQ ID NO: 2, having α-amylase activity, and having deletion of amino acid residues at two or more positions selected from the group consisting of positions R178, G179, T180, and G181 of the amino acid sequence of SEQ ID NO: 2;
   (B') a protein consisting of an amino acid sequence having at least 90% identity to the amino acid sequence of SEQ ID NO: 4, having α-amylase activity, and having deletion of amino acid residues at two or more positions selected from the group consisting of positions R178, G179, T180, and G181 of the amino acid sequence of SEQ ID NO: 4;
   (C') a protein consisting of an amino acid sequence having at least 90% identity to the amino acid sequence of SEQ ID NO: 6, having α-amylase activity, and having deletion of amino acid residues at two or more positions selected from the group consisting of positions R178, G179, T180, and G181 of the amino acid sequence of SEQ ID NO: 6; and
   (D') a protein consisting of an amino acid sequence having at least 90% identity to the amino acid sequence of SEQ ID NO: 8, having α-amylase activity, and having deletion of amino acid residues at two or more positions selected from the group consisting of positions R178, G179, T180, and G181 of the amino acid sequence of SEQ ID NO: 8.
<15> The cleaning composition according to <13> or <14>, wherein the protein having α-amylase activity is a protein having a relative cleaning performance of 1 or more, preferably 1.1 or more, more preferably 1.2 or more, even more preferably 1.3 or more, even more preferably 1.5 or more, even more preferably 1.7 or more, and even more preferably 1.9 or more, with respect to an amylase consisting of the amino acid sequence of SEQ ID NO: 12.
<16> The cleaning composition according to any one of <13> to <15>, wherein the protein having protease activity is a protein having at least 70% identity to the amino acid sequence of SEQ ID NO: 42, 43, 44, 45, 46, 47, or 48, and having protease activity.
<17> The cleaning composition according to any one of <13> to <16>, which is a clothing cleaning agent or a dishwashing cleaning agent.
<18> The cleaning composition according to <17>, which is a clothing cleaning agent for laundry or a dishwashing cleaning agent for hand washing or an automatic dishwasher.
<19> The cleaning composition according to <17> or <18>, which is a powder or a liquid.
<20> The cleaning composition according to any one of <17> to <19>, which is used at a low temperature.
<21> The cleaning composition according to <20>, which is used at 40°C or lower, 35°C or lower, 30°C or lower, or 25°C or lower, and 5°C or higher, 10°C or higher, or 15°C or higher, or used at from 5 to 40°C, from 10 to 35°C, from 15 to 30°C, or from 15 to 25°C.
<22> The cleaning composition according to <17>, which is used in low-temperature (from 15 to 30°C) cleaning in laundries or low-temperature (from 15 to 30°C) cleaning by the automatic dishwasher.
<23> The cleaning composition according to any one of <14> to <22>, wherein the deletion of amino acid residues at two or more positions selected from the group consisting of positions R178, G179, T180, and G181 is R178Δ+T180Δ, G179Δ+T180Δ, R178Δ+G179Δ, R178Δ+G181Δ, or G179Δ+G181Δ, and preferably R178Δ+T180Δ.

### Examples

### (1) Construction of amylase expression plasmid

PCR was performed using the DE0178 gene (SEQ ID NO: 1) obtained by artificial gene synthesis as a template, a primer pair DE0178_fw/DE0178_rv (SEQ ID NOs: 19 and 20) and PrimeSTAR Max Premix (Takara Bio Inc.). Using the plasmid pHY-S237 described in Example 7 of WO 2006/068148 A1 as a template, PCR was similarly performed using a primer pair S237t_fw/S237s_rv (SEQ ID NOs: 17 and 18). Using each of the PCR products, the In-Fusion reaction was performed in accordance with the protocol of the In-Fusion, HD Cloning kit (Clontech). Using the In-Fusion reaction solution, *Bacillus subtilis* was transformed to construct plasmid pHY-DE0178. Similarly, for RU2C, BCGAmy, YR288, AP1378, AA560, SP722, and CspAmy2, genes obtained by artificial gene synthesis (SEQ ID NOs: 3, 5, 7, 9, 11, 13, and 15) were each used as a template, and PCR and In-Fusion reaction were performed using a primer pair RU2C_fw/RU2C_rv (SEQ ID NOs: 21 and 22), BCGAmy_fw/BCGAmy_rv (SEQ ID NOs: 23 and 24), YR288_fw/YR288_rv (SEQ ID NOs: 25 and 26), AP1378_fw/AP1378_rv (SEQ ID NOs: 27 and 28), AA560_fw/AA560_rv (SEQ ID NOs: 29 and 30), SP722_fw/SP722_rv (SEQ ID NOs: 31 and 32), or CspAmy2_fw/CspAmy2_rv (SEQ ID NOs: 33 and 34). The In-Fusion reaction solutions were transformed into *Bacillus subtilis* to construct plasmids pHY-RU2C, pHY-BCGAmy, pHY-YR288, pHY-AP1378, pHY-AA560, pHY-SP722, and pHY-CspAmy2.

### (2) Transformation

The host used was *Bacillus subtilis* 168 strain (*Bacillus subtilis* Marburg No. 168 strain: Nature, 390, 1997, p. 249). The *Bacillus subtilis* 168 strain was inoculated in 1 mL of LB culture medium and cultured by shaking at 30°C and 200 rpm overnight. 10 µL of the culture was inoculated in 1 mL of fresh LB culture medium and cultured at 37°C and 200 rpm for 3 hours. The culture was centrifuged, and pellets were collected. 500 µL of SMMP (0.5 M sucrose, 20 mM disodium malate, 20 mM magnesium chloride hexahydrate, and 35% (w/v) antibiotic medium 3 (Difco)) containing 4 mg/mL lysozyme (SIGMA) was added to the pellets, followed by incubation at 37°C for 1 hour. Next, the pellets were collected by centrifugation and suspended in 400 µL of SMMP. 33 µL of the suspension was mixed with DNA, and 100 µL of 40% PEG was further added, followed by stirring. Further, 350 µL of SMMP was added, followed by shaking at 30°C for 1 hour. 200 µL of the resulting liquid was smeared to DM3 regeneration agar culture medium (0.8% agar (FUJIFILM Wako Pure Chemical Corporation), 0.5% disodium succinate hexahydrate, 0.5% casamino acids technical (Difco), 0.5% yeast extract, 0.35% monopotassium phosphate, 0.15% dipotassium phosphate, 0.5% glucose, 0.4% magnesium chloride hexahydrate, 0.01% bovine serum albumin (SIGMA), 0.5% carboxymethyl cellulose, 0.005% trypan blue (Merck), and an amino acid mixed solution (tryptophan, lysine, and methionine, each 10 µg/mL); % denotes (w/v)%) containing tetracycline (15 µg/mL, SIGMA), followed by incubation at 30°C for 3 days, and the formed colonies were obtained.

### (3) Enzyme production culture

The recombinant *Bacillus subtilis* colonies obtained in (2) were inoculated in a 96-deep-well plate into which 300 µL of LB culture medium supplemented with 15 ppm tetracycline was dispensed, and then cultured at 30°C at 210 rpm overnight. Next day, 6 µL of the culture was inoculated in a 96-deep-well plate into which 100 µL of 2xL-maltose culture medium (2% tryptone, 1% yeast extract, 1% NaCl, 7.5% maltose, 7.5 ppm manganese sulfate pentahydrate, 0.04% calcium chloride dihydrate, and 15 ppm tetracycline; % denotes (w/v)%) was dispensed, and cultured at 30°C at 210 rpm for 2 days. Then, the culture supernatant containing the enzyme produced from the bacterial cell was collected by centrifugation.

### (4) Protein concentration measurement

For the protein concentration measurement, Protein Assay Rapid Kit Wako II (FUJIFILM Wako Pure Chemical Corporation) was used. The protein concentration of a culture supernatant of a strain introduced with pHY300PLK (Takara Bio Inc.) having no amylase expression cassette was used as a blank to calculate the amylase concentration of the culture supernatant.

### (5) Amylolytic activity measurement

The amylolytic activity of each culture supernatant and Termamyl (SIGMA, A4862) was measured using Phadebas (Phadebas AB). Phadebas is a tablet made of insoluble starch covalently bonded to a blue dye. The watersoluble blue dye is released in association with amylolysis by α-amylase. The concentration of the blue dye measured by the absorbance at 620 nm is proportional to the amylase activity in the sample.

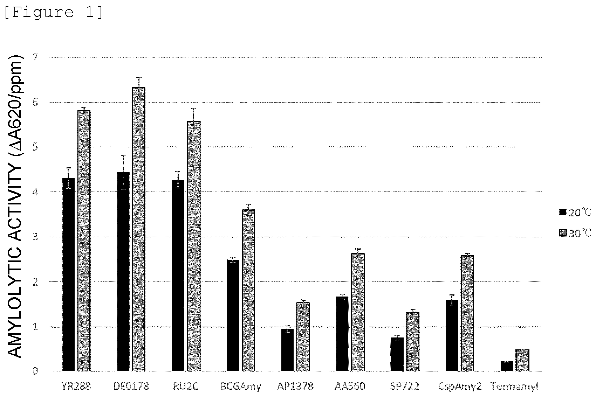

One substrate tablet was suspended per 5 mL of 1/15 M phosphate buffer (pH: 7.4). 500 µL of the substrate suspension was dispensed into a 96-deep-well plate. An enzyme solution suitably diluted with 1/15 M phosphate buffer (pH: 7.4) was added, followed by mixing. After standing still at 20°C or 30°C for 30 minutes, 250 µL of a 10% aqueous citric acid solution was added to terminate the reaction. After centrifugation at 3,000 rpm for 20 minutes, 100 µL of the supernatant was transferred to a new 96-well plate, and the absorbance at 620 nm was measured. It was confirmed that each measured value was within the linear range of activity measurement. By subtracting the value of the blank (enzyme-free), amylolytic activity ΔA620 was calculated, and the resulting value was further divided by the added amylase concentration to determine specific activity ΔA620/ppm (Fig. 1).

DE0178, RU2C, BCGAmy, and YR288 showed significantly higher amylolytic activity at 20°C and 30°C in comparison with the previously reported amylases for a cleaning agent (AP1378, AA560, SP722, CspAmy2, and Termamyl).

### (6) Phylogenetic tree

A phylogenetic tree was constructed using the amino acid sequences of the mature regions of the amylases DE0178, RU2C, BCGAmy, and YR288 which maintained high activity at low temperatures, and the existing amylases for a cleaning agent AP1378, AA560, SP722, CspAmy2, BAA (SEQ ID NO: 35), BLA (SEQ ID NO: 36), LABM (SEQ ID NO: 37), SP707 (SEQ ID NO: 38), TS23 (SEQ ID NO: 39), Termamyl (SEQ ID NO: 40), and AAI10 (SEQ ID NO: 41). Multiple alignment was performed using Genetyx by clustalW, and the phylogenetic tree was constructed by the neighbor-joining method (NJ method).

As a result, the amylases DE0178, RU2C, BCGAmy, and YR288 which maintained high activity at low temperatures formed different groups from the existing cleaning agent amylases (Fig. 2).

### (7) Detergency evaluation

A CS-26 stained cloth cut into a circular shape with a diameter of 5.5 mm was obtained from CFT. Two CS-26 circular stained cloths were inserted into each well of a 96-well assay plate, and 200 µL of Attack Zero (Kao Corporation) diluted 3,000-fold with tap water was added to each well. 10 µL of a suitably diluted enzyme solution was added to each well, and the plate was sealed and shaken at 20°C using a Cute Mixer at 1,200 rpm for 15 minutes. After the completion of cleaning, 100 µL of the cleaning liquid was transferred to a new 96-well assay plate, and the absorbance at 488 nm was measured. A blank was prepared by adding tap water in place of the enzyme solution, and the difference from the blank ΔA488 was determined as detergency.

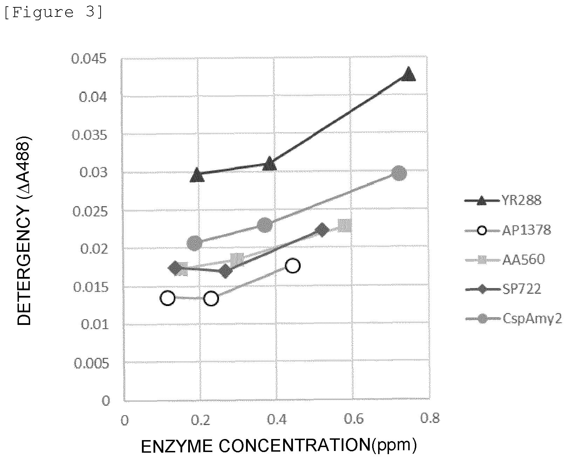

The amylase YR288, which maintained high activity at low temperatures, showed significantly higher detergency at low temperatures in comparison with the previously reported amylases for a cleaning agent (AP1378, AA560, SP722, and CspAmy2) (Fig. 3).

### (8) Construction of amylase mutant expression plasmid

A forward primer having 15 bases of a sequence complementary to a reverse primer at the 5'-terminal and containing a mutant sequence, and a reverse primer having a base just before the mutant sequence at the 5'-terminal were used as a mutagenesis primer pair. The amylase expression plasmid produced in Example (1) was used as a template, and PCR was performed by using the mutagenesis primer pair. With the PCR product, *Bacillus subtilis* was transformed by the protoplast method to obtain a transformant retaining the target mutant expression plasmid.

### (9) Evaluation of mutant with deletion of two amino acids at positions from 178 to 181 of YR288

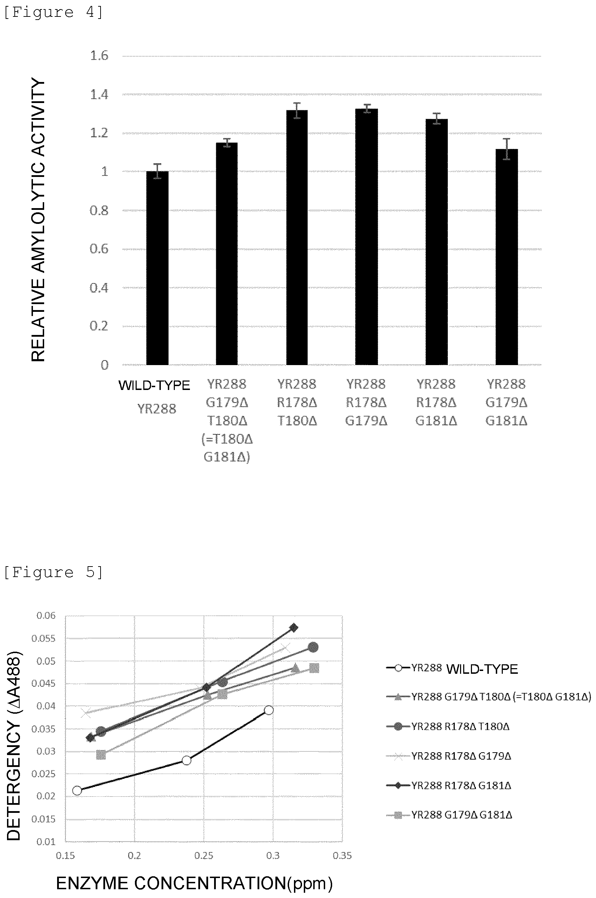

The mutants shown in Fig. 4 were constructed by the method described in Example (8) using YR288 (SEQ ID NO: 8) as a parent polypeptide.

The amylolytic specific activity ΔA620/ppm of each mutant at 20°C was determined by the method described in Example (5), and the resulting value was divided by the value of wild-type YR288 to determine the relative specific activity (Fig. 4). The amylolytic activity of YR288 at low temperatures was further enhanced by deleting any two residues out of R178, G179, T180, and G181.

The detergency of the mutants was evaluated by the method described in Example (7). The detergency of YR288 was further enhanced by deleting any two residues out of R178, G179, T180, and G181 (Fig. 5).

### (10) Evaluation of mutant with deletion of two amino acids at positions from 178 to 181 of BCGAmy

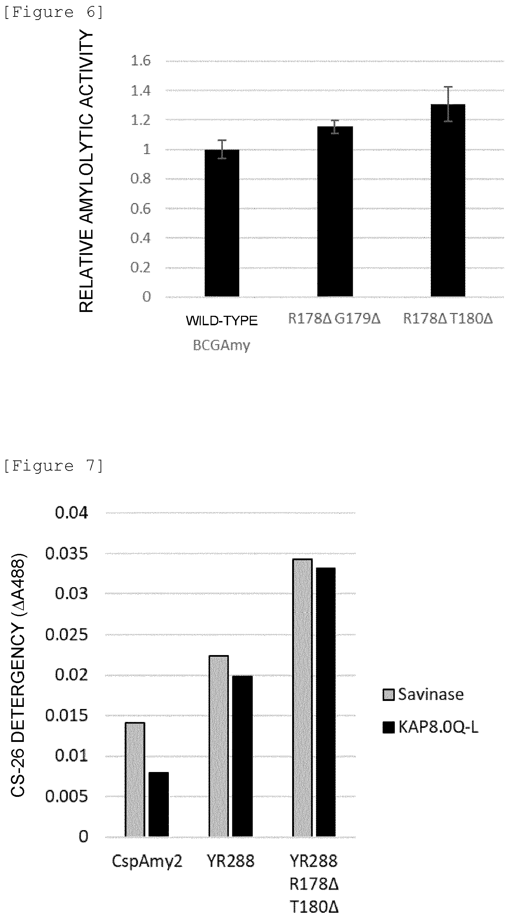

The mutants shown in Fig. 6 were constructed by the method described in Example (8) using BCGAmy (SEQ ID NO: 6) as a parent polypeptide.

The amylolytic specific activity ΔA620/ppm of each mutant at 20°C was determined by the method described in Example (5), and the resulting value was divided by the value of wild-type BCGAmy to determine the relative specific activity (Fig. 6). The amylolytic activity of BCGAmy at low temperatures was further enhanced by deleting two residues R178 and G179, or two residues R178 and T180.

### (11) Relative cleaning performance of y288 and mutant with deletion of two amino acids of YR288 with respect to AA560

A CS-26 stained cloth cut into a circular shape with a diameter of 5.5 mm was obtained from CFT. Two CS-26 circular stained cloths were inserted into each well of a 96-well assay plate (3881-096, IWAKI), and 200 µL of a model cleaning liquid (200 ppm sodium linear alkylbenzene sulfonate (195-07682, Wako) and 20 mM Tris HCl aqueous solution (pH: 7.5)) was added to each well. 10 µL of each of the enzyme solutions shown in Table 1 diluted with ion-exchange water to 4 ppm was added to each well, and the plate was sealed and shaken at 20°C using a Cute Mixer (CM-1000, EYELA) at 1,200 rpm for 15 minutes. After the completion of cleaning, 100 µL of the cleaning liquid was transferred to a new 96-well assay plate, and the absorbance at 488 nm was measured. A blank was prepared by adding ion-exchange water in place of the enzyme solution, and the difference from the blank ΔA488 was determined. The ΔA488 of each enzyme was divided by the ΔA488 of AA560 to determine the relative cleaning performance with respect to AA560 (Table 1).

**[Table 1]**

| | Relative cleaning performance for AA560 |
|---|---|
| AA560 | 1.0 |
| CspAmy2 | 0.8 |
| YR288 | 1.3 |
| YR288 R178Δ T180Δ | 1.9 |

### (12) Cleaning performance of cleaning composition containing amylase and protease

A CS-26 stained cloth cut into a circular shape with a diameter of 5.5 mm was obtained from CFT, and an EMPA117 stained cloth was obtained from EMPA. The proteases used were Savinase (SIGMA, P3111) and KAP8.0Q-L (Kao Corporation). The CS-26 stained cloth or the EMPA117 stained cloth was inserted into each well of a 96-well assay plate, and 200 µL of Attack Zero (Kao Corporation) diluted 3,000-fold with tap water (treated at 90°C for 5 hours) or a model cleaning liquid (200 ppm sodium linear alkylbenzene sulfonate (195-07682, Wako) and 20 mM Tris HCl aqueous solution (pH: 7.5)) was added to each well. 10 µL of an amylase solution diluted to 4 ppm and 10 µL of a protease solution diluted to 4 ppm were added to each well, and the plate was sealed and shaken at 20°C using a Cute Mixer at 1,200 rpm for 15 minutes. After the completion of cleaning, 100 µL of the cleaning liquid was transferred to a new 96-well assay plate, and the absorbance at 488 nm for the CS-26 stained cloth and the absorbance at 660 nm for the EMPA117 stained cloth were measured. A blank was prepared by adding ion-exchange water in place of the amylase solution, and the difference of absorbance from the blank ΔA488 was determined as amylase detergency.

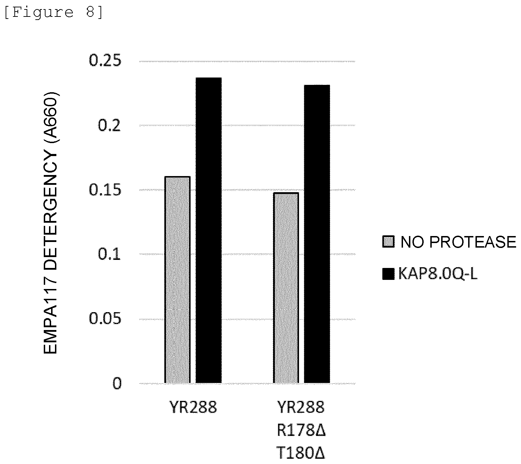

Fig. 7 shows the detergency against the CS-26 stained cloth, and Fig. 8 shows the detergency against the EMPA117 stained cloth. The cleaning compositions containing the amylases which maintained high activity at low temperatures (YR288 and YR288 R178Δ T180Δ) and protease exhibited superior detergency at low temperatures in comparison with the cleaning compositions containing the conventional amylases and protease.

## Claims

1. A cleaning composition comprising one or more proteins selected from the group consisting of the following (A), (B), (C), and (D):
(A) a protein consisting of an amino acid sequence having at least 90% identity to the amino acid sequence of SEQ ID NO: 2, and having α-amylase activity;
(B) a protein consisting of an amino acid sequence having at least 90% identity to the amino acid sequence of SEQ ID NO: 4, and having α-amylase activity;
(C) a protein consisting of an amino acid sequence having at least 90% identity to the amino acid sequence of SEQ ID NO: 6, and having α-amylase activity; and
(D) a protein consisting of an amino acid sequence having at least 90% identity to the amino acid sequence of SEQ ID NO: 8, and having α-amylase activity.

2. The cleaning composition according to claim 1, further comprising a protein having protease activity.

3. The cleaning composition according to claim 1 or 2, wherein the protein is one or more selected from the group consisting of the following (A'), (B'), (C'), and (D'):
(A') a protein consisting of an amino acid sequence having at least 90% identity to the amino acid sequence of SEQ ID NO: 2, having α-amylase activity, and having deletion of amino acid residues at two or more positions selected from the group consisting of positions R178, G179, T180, and G181 of the amino acid sequence of SEQ ID NO: 2;
(B') a protein consisting of an amino acid sequence having at least 90% identity to the amino acid sequence of SEQ ID NO: 4, having α-amylase activity, and having deletion of amino acid residues at two or more positions selected from the group consisting of positions R178, G179, T180, and G181 of the amino acid sequence of SEQ ID NO: 4;
(C') a protein consisting of an amino acid sequence having at least 90% identity to the amino acid sequence of SEQ ID NO: 6, having α-amylase activity, and having deletion of amino acid residues at two or more positions selected from the group consisting of positions R178, G179, T180, and G181 of the amino acid sequence of SEQ ID NO: 6; and
(D') a protein consisting of an amino acid sequence having at least 90% identity to the amino acid sequence of SEQ ID NO: 8, having α-amylase activity, and having deletion of amino acid residues at two or more positions selected from the group consisting of positions R178, G179, T180, and G181 of the amino acid sequence of SEQ ID NO: 8.

4. The cleaning composition according to any one of claims 1 to 3, wherein the protein is a protein having a relative cleaning performance of 1 or more with respect to an amylase consisting of the amino acid sequence of SEQ ID NO: 12.

5. The cleaning composition according to any one of claims 2 to 4, wherein the protein having protease activity is a protein having at least 70% identity to the amino acid sequence of SEQ ID NO: 42, 43, 44, 45, 46, 47, or 48, and having protease activity.

6. The cleaning composition according to any one of claims 1 to 5, which is a clothing cleaning agent or a dishwashing cleaning agent.

7. The cleaning composition according to claim 6, which is a clothing cleaning agent for laundry or a dishwashing cleaning agent for hand washing or an automatic dishwasher.

8. The cleaning composition according to claim 6 or 7, which is a powder or a liquid.

9. The cleaning composition according to any one of claims 6 to 8, which is used at a low temperature.

10. The cleaning composition according to claim 9, which is used at a temperature of from 5 to 40°C.

11. A protein selected from the group consisting of the following (A'), (B'), (C'), and (D'):
(A') a protein consisting of an amino acid sequence having at least 90% identity to the amino acid sequence of SEQ ID NO: 2, having α-amylase activity, and having deletion of amino acid residues at two or more positions selected from the group consisting of positions R178, G179, T180, and G181 of the amino acid sequence of SEQ ID NO: 2;
(B') a protein consisting of an amino acid sequence having at least 90% identity to the amino acid sequence of SEQ ID NO: 4, having α-amylase activity, and having deletion of amino acid residues at two or more positions selected from the group consisting of positions R178, G179, T180, and G181 of the amino acid sequence of SEQ ID NO: 4;
(C') a protein consisting of an amino acid sequence having at least 90% identity to the amino acid sequence of SEQ ID NO: 6, having α-amylase activity, and having deletion of amino acid residues at two or more positions selected from the group consisting of positions R178, G179, T180, and G181 of the amino acid sequence of SEQ ID NO: 6; and
(D') a protein consisting of an amino acid sequence having at least 90% identity to the amino acid sequence of SEQ ID NO: 8, having α-amylase activity, and having deletion of amino acid residues at two or more positions selected from the group consisting of positions R178, G179, T180, and G181 of the amino acid sequence of SEQ ID NO: 8.

12. The protein according to claim 11, which has a relative cleaning performance of 1 or more with respect to an amylase consisting of the amino acid sequence of SEQ ID NO: 12.
